# EUROPÄISCHE PATENTSCHRIFT

(11) **EP 0 361 532 B1**
(45) Veröffentlichungstag und Bekanntmachung des Hinweises auf die Patenterteilung: **06.04.1994**
(21) Anmeldenummer: 89119488.8
(22) Anmeldetag: 16.12.1986
(51) Int. Cl.: C07C 43/115, C09K 19/30, G02F 1/13, C07C 43/164, C07C 43/21, C07C 43/30

(54) **Ethanderivate**
Ethane derivatives
Dérivés de l'éthane

(30) Priorität: 20.01.1986 DE 3601452
(43) Veröffentlichungstag der Anmeldung: 04.04.1990
(62) Teilanmeldung aus: 87901266.4
(73) Patentinhaber: MERCK PATENT GmbH, D-64271 Darmstadt (DE)
(72) Erfinder: Wächtler, Andreas, Dr., D-6103 Griesheim (DE); Krause, Joachim, Dr., D-6110 Dieburg (DE); Eidenschink, Rudolf, Dr., D-6501 Bodenheim (DE); Scheuble, Bernhard, Dr., Yokohama-Shi Kanagawa 231 (JP); Hittich, Reinhard, Dr., D-6101 Modautal 1 (DE)

(56) Entgegenhaltungen:
- EP-A- 0 056 113
- EP-A- 0 058 981

## Beschreibung

Die Erfindung betrifft neue Ethanderivate ausgewählt aus den Formeln 1a und 1c, 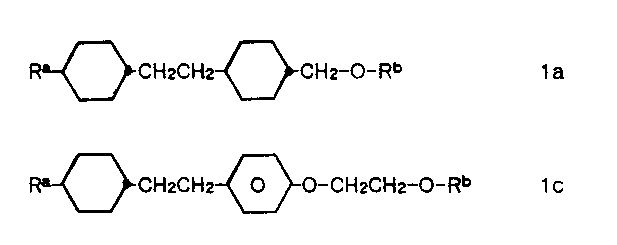
worin
- R^{a}: geradkettiges Alkyl mit 1 - 8 C-Atomen, und
- R^{b}: geradkettiges Alkyl mit 1 - 8 C-Atomen, worin auch eine oder zwei nicht benachbarte und jeweils nicht endständige CH₂-Gruppe(n) durch -O- ersetzt sein können,
bedeuten, sowie flüssigkristalline Phasen enthaltend Verbindungen der Formel 1a oder 1c und deren Verwendung als Komponenten flüssigkristalliner Phasen.

Die Verbindungen der Formeln 1a und 1c besitzen eine hohe chemische Stabilität und nur geringe Leitfähigkeit. Sie sind farblos und gut mischbar mit allen gebräuchlichen Flüssigkristallen. Ihr Einsatz führt zu breiteren Mesophasenbereichen und verringerter Viskosität. Die erfindungsgemäßen Verbindungen der Formeln 1a und 1c eignen sich somit sehr gut für flüssigkristalline Phasen für elektrooptische Anzeigevorrichtungen, wie beispielsweise TN-Zellen oder Guest-Host-Zellen.

Die Verbindungen der Formel 1a werden von der breiten, allgemeinen Formel der EP-A-0 058 981 zwar umfaßt, jedoch werden dort keine Verbindungen beschrieben, worin zwei 6-gliedrige Ringe über eine Ethylengruppe verbunden sind. Weiterhin konnte der Fachmann der EP-A-0 058 981 keinen Hinweis entnehmen, daß die erfindungsgemäßen Verbindungen zu flüssigkristallinen Medien mit verbesserten Eigenschaften für elektooptische Anzeigen führen.

Gegenstand der Erfindung sind demnach Ethanderivate ausgewählt aus den Formeln 1a und 1c, 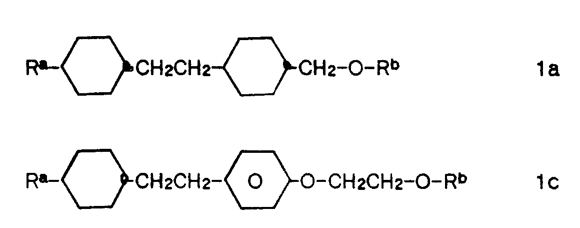
worin
- R^{a}: geradkettiges Alkyl mit 1 - 8 C-Atomen, und
- R^{b}: geradkettiges Alkyl mit 1 - 8 C-Atomen, worin auch eine oder zwei nicht benachbarte und jeweils nicht endständige CH₂-Gruppe(n) durch -O- ersetzt sein können,
bedeuten.

Weiterhin Gegenstand der Erfindung ist die Verwndung der Verbindungen der Formeln 1a und 1c als Komponenten flüssigkristalliner Phasen.

Flüssigkristalline Phasen mit mindestens zwei flüssigkristallinen Komponenten, dadurch gekennzeichnet, daß sie mindestens eine Verbindung der Formeln 1a oder 1c enthalten, sind ebenfalls Gegenstand der Erfindung, sowie ein Flüssigkristall-Anzeigeelement, dadurch gekennzeichnet, daß es eine flüssigkristalline Phase nach Anspruch 8 enthält, insbesondere, ein elektrooptisches Anzeigeelement, dadurch gekennzeichnet, daß es als Dielektrikum eine flüssigkristalline Phase nach Anspruch 8 enthält.

R^{a} bedeutet vorzugsweise geradkettiges Alkyl mit zwei bis 5 C-Atomen.

R^{b} ist in den Verbindungen der Teilformeln 1a vorzugsweise geradkettiges Alkyl mit 1 bis 5, insbesondere mit 1 bis 3, C-Atomen. In den Verbindungen der Teilformel 1c bedeutet R^{b} vorzugsweise geradkettiges Alkyl mit 1 bis 3 C-Atomen, insbesondere bevorzugt Methyl oder Ethyl. Falls R^{b} eine Alkylgruppe bedeutet, worin auch eine oder zwei nicht benachbarte und jeweils nicht endständige CH₂-Gruppen durch -O-ersetzt sind, sind Reste der Formel -CH₂-CH₂-O-Alkyl oder -CH₂-CH₂-O-CH₂CH₂-O-Alkyl bevorzugt. Verbindungen der Formeln 1a/c mit derartigen Resten sind ebenfalls bevorzugt.

Die Verbindungen der Formeln 1a/c können durch Veretherung entsprechender Hydroxyverbindungen hergestellt werden. Entsprechende Hydroxyverbindungen sind entweder bekannt oder in an sich bekannter Weise herstellbar.

Zur Veretherung kann die Hydroxyverbindung zunächst in ein entsprechendes Metallderivat, z.B. durch Behandeln mit Na, K, NaH, KH oder K₂CO₃ in das entsprechende Alkalimetallalkoholat bzw. -phenolat umgewandelt werden. Dieses kann dann z.B. mit einem Alkylhalogenid oder -sulfonat oder einem Dialkylsulfat umgesetzt werden, zweckmäßig in Gegenwart eines inerten Lösungsmittels wie Aceton, 1,2-Dimethoxyethan, THF, Dimethylformamid oder Dimethylsulfoxid bei Temperaturen zwischen etwa 20 und 100°.

Die erfindungsgemäßen flüssigkristallinen Phasen bestehen aus 2 bis 25, vorzugsweise 3 bis 15 Komponenten, darunter mindestens einer Verbindung der Formeln 1a/c
Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel VI charakterisieren,

R³-L-G-E-R⁴ VI

worin L und E je ein carbo- oder heterocyclisches Ringsystem aus der aus 1,4-disubstituierten Benzol- und Cyclohexanringen, 4,4′-disubstituierten Biphenyl-, Phenylcyclohexan- und Cyclohexylcyclohexansystemen, 2,5-disubstierten Pyrimidin- und 1,3-Dioxanringen, 2,6-disubstituiertem Naphthalin, Di- und Tetrahydronaphthalin, Chinazolin und Tetrahydrochinazolin gebildeten Gruppe

| | | |
|---|---|---|
| G | -CH=CH- | -N(O)=N- |
| | -CH=CY | -CH=N(O)- |
| | -C≡C- | -CH₂-CH₂- |
| | -CO-O- | -CH₂-O- |
| | -CO-S- | -CH₂-S- |
| | -CH=N- | -COO-Phe-COO |

oder eine C-C-Einfachbindung, Y Halogen, vorzugsweise Chlor, oder -CN, und R³ und R⁴ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, NO₂, CF₃, F, Cl oder Br bedeuten.

Bei den meisten dieser Verbindungen sind R³ und R⁴ voneinander verschieden, wobei einer dieser Reste meist meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach an sich bekannten Methoden herstellbar.

Die erfindungsgemäßen flüssigkristallinen Phase enthalten etwa 0,1 bis 99, vorzugsweise 1 bis 30 %, einer oder mehrerer Verbindungen der Formel 1 .

Die Herstellung der erfindungsgemäßen flüssigkristallinen Phasen erfolgt in an sich üblicher Weise. In der Regel werden die Komponenten ineinander gelöst, zweckmäßig bei erhöhter Temperatur.

Durch geeignete Zustände können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vg. z.B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase ein und reinigt das Produkt durch Kristallisation, Destillation und/oder Chromatographie.

### Beispiel 1

Zu einem Gemisch von 22 g n-Butyljodid und 4,8 g NaH in 250 ml THF tropft man bei 50° unter Rühren eine Lösung von 25,7 g 1-(trans-4-n-Propylcyclohexyl)-2-(trans-4-hydroxymethylcyclohexyl)-ethan [erhältlich durch Reduktion der aus 1-(trans-4-n-Propylcyclohexyl)-2-(trans-4-cyancyclohexyl)-ethan (DE-OS 31 00 142) durch Verseifung zugänglichen Carbonsäure mit Lithiumaluminiumhydrid] in 200 ml THF und rührt noch 4 Stunden bei 50°. Nach üblicher Aufarbeitung erhält man 1-(trans-4-n-Propylcyclohexyl)-2-[trans-4-(2-oxa-hexyl)-cyclohexyl]-ethan.

Analog werden hergestellt:
1-(trans-4-Propylcyclohexyl)-2-[trans-4-(2-oxapropyl)-cyclohexyl]-ethan
1-(trans-4-Propylcyclohexyl)-2-[trans-4-(2-oxabutyl)-cyclohexyl]-ethan
1-(trans-4-Propylcyclohexyl)-2-[trans-4-(2-oxapentyl)-cyclohexyl]-ethan
1-(trans-4-Propylcyclohexyl)-2-[trans-4-(2-oxaheptyl)-cyclohexyl]-ethan
1-(trans-4-Ethylcyclohexyl)-2-[trans-4-(2-oxapropyl)-cyclohexyl]-ethan
1-(trans-4-Ethylcyclohexyl)-2-[trans-4-(2-oxabutyl)-cyclohexyl]-ethan
1-(trans-4-Ethylcyclohexyl)-2-[trans-4-(2-oxapentyl)-cyclohexyl]-ethan
1-(trans-4-Ethylcyclohexyl)-2-[trans-4-(2-oxahexyl)-cyclohexyl]-ethan
1-(trans-4-Ethylcyclohexyl)-2-[trans-4-(2-oxaheptyl)-cyclohexyl]-ethan
1-(trans-4-Butylcyclohexyl)-2-[trans-4-(2-oxapropyl)-cyclohexyl]-ethan
1-(trans-4-Butylcyclohexyl-2-[trans-4-(2-oxabutyl)-cyclohexyl]-ethan
1-(trans-4-Butylcyclohexyl)-2-[trans-4-(2-oxapentyl)-cyclohexyl]-ethan
1-(trans-4-Butylcyclohexyl)-2-[trans-4-(2-oxahexyl)-cyclohexyl]-ethan
1-(trans-4-Butylcyclohexyl)-2-[trans-4-(2-oxaheptyl)-cyclohexyl]-ethan
1-(trans-4-Pentylcyclohexyl)-2-[trans-4-(2-oxapropyl)-cyclohexyl]-ethan
1-(trans-4-Pentylcyclohexyl)-2-[trans-4-(2-oxabutyl)-cyclohexyl]-ethan
1-(trans-4-Pentylcyclohexyl)-2-[trans-4-(2-oxapentyl)-cyclohexyl]-ethan
1-(trans-4-Pentylcyclohexyl)-2-[trans-4-(2-oxahexyl)-cyclohexyl]-ethan
1-(trans-4-Pentylcyclohexyl)-2-[trans-4-(2-oxaheptyl)-cyclohexyl]-ethan
1-(trans-4-Propylcyclohexyl)-2-[trans-4-(2,5-dioxahexyl)-cyclohexyl]-ethan
1-(trans-4-Propylcyclohexyl)-2-[trans-4-(2,5-dioxaheptyl)-cyclohexyl]-ethan
1-(trans-4-Ethylcyclohexyl)-2-[trans-4-(2,5-dioxahexyl)-cyclohexyl]-ethan
1-(trans-4-Ethylcyclohexyl)-2-[trans-4-(2,5-dioxaheptyl)-cyclohexyl]-ethan
1-(trans-4-Butylcyclohexyl)-2-[trans-4-(2,5-dioxahexyl)-cyclohexyl]-ethan
1-(trans-4-Butylcyclohexyl)-2-[trans-4-(2,5-dioxaheptyl)-cyclohexyl]-ethan
1-(trans-4-Pentylcyclohexyl)-2-[trans-4-(2,5-dioxahexyl)-cyclohexyl]-ethan
1-(trans-4-Pentylcyclohexyl)-2-[trans-4-(2,5-dioxaheptyl)-cyclohexyl]-ethan

### Beispiel 2

Ein Gemisch von 26 g p-[2-(trans-4-n-Butylcyclohexyl)-ethyl]-phenol (DE-OS 32 01 721), 6,9 g K₂CO₃, 16,5 g 2-Bromethylmethylether und 250 ml DMF wird unter Rühren 16 Stunden auf 80° erhitzt, dann abgekühlt und wie üblich aufgearbeitet. Man erhält 1-(trans-4-n-Butylcyclohexyl)-2-[p-(1,4-dioxapentyl)-phenyl]-ethan.

Analog werden hergestellt:
1-(trans-4-Butylcyclohexyl)-2-[p-(1,4-dioxahexyl)-phenyl)-ethan
1-(trans-4-Butylcyclohexyl)-2-[p-(1,4-dioxaheptyl)-phenyl)-ethan
1-(trans-4-Butylcyclohexyl)-2-[p-(1,3-dioxapentyl)-phenyl)-ethan
1-(trans-4-Ethylcyclohexyl)-2-[p-(1,4-dioxahexyl)-phenyl)-ethan
1-(trans-4-Ethylcyclohexyl)-2-[p-(1,4-dioxaheptyl)-phenyl)-ethan
1-(trans-4-Propylcyclohexyl)-2-[p-(1,4-dioxahexyl)-phenyl)-ethan
1-(trans-4-Propylcyclohexyl)-2-[p-(1,4-dioxaheptyl)-phenyl)-ethan
1-(trans-4-Pentylcyclohexyl)-2-[p-(1,4-dioxahexyl)-phenyl)-ethan
1-(trans-4-Pentylcyclohexyl)-2-[p-(1,4-dioxaheptyl)-phenyl)-ethan

## Patentansprüche

1. Ethanderivate ausgewählt aus den Formeln 1a und 1c, 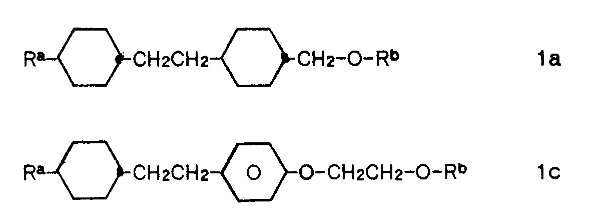 worin
R^{a} geradkettiges Alkyl mit 1 - 8 C-Atomen, und
R^{b} geradkettiges Alkyl mit 1 - 8 C-Atomen, worin auch eine oder zwei nicht benachbarte und jeweils nicht endständige CH₂-Gruppe(n) durch -O- ersetzt sein können,
bedeuten.

2. Ethanderivate nach Anspruch 1, wobei
R^{a} geradkettiges Alkyl mit 2 - 5 C-Atomen bedeutet.

3. Ethanderivate der Teilformel 1a nach Anspruch 1 oder 2, wobei
R^{b} geradkettiges Alkyl mit 1 - 5, insbesondere mit 1 - 3 C-Atomen bedeutet.

4. Ethanderivate der Teilformel 1c nach Anspruch 1 oder 2, wobei
R^{b} geradkettiges Alkyl mit 1 - 3 C-Atomen, insbesondere Methyl oder Ethyl bedeutet.

5. Ethanderivate der Teilformel 1a nach Anspruch 1, 2 oder 3 ausgewählt aus den folgenden Verbindungen:
1-(trans-4-Propylcyclohexyl)-2-[trans-4-(2-oxapropyl)-cyclohexyl]-ethan
1-(trans-4-Propylcyclohexyl)-2-[trans-4-(2-oxabutyl)-cyclohexyl]-ethan
1-(trans-4-Propylcyclohexyl)-2-[trans-4-(2-oxapentyl)-cyclohexyl]-ethan
1-(trans-4-n-Propylcyclohexyl)-2-[trans-4-(2-oxahexyl)-cyclohexyl]-ethan
1-(trans-4-Propylcyclohexyl)-2-[trans-4-(2-oxaheptyl)-cyclohexyl]-ethan
1-(trans-4-Ethylcyclohexyl)-2-[trans-4-(2-oxapropyl)-cyclohexyl]-ethan
1-(trans-4-Ethylcyclohexyl)-2-[trans-4-(2-oxabutyl)-cyclohexyl]-ethan
1-(trans-4-Ethylcyclohexyl)-2-[trans-4-(2-oxapentyl)-cyclohexyl]-ethan
1-(trans-4-Ethylcyclohexyl)-2-[trans-4-(2-oxahexyl)-cyclohexyl]-ethan
1-(trans-4-Ethylcyclohexyl)-2-[trans-4-(2-oxaheptyl)-cyclohexyl]-ethan
1-(trans-4-Butylcyclohexyl)-2-[trans-4-(2-oxapropyl)-cyclohexyl]-ethan
1-(trans-4-Butylcyclohexyl)-2-[trans-4-(2-oxabutyl)-cyclohexyl]-ethan
1-(trans-4-Butylcyclohexyl)-2-[trans-4-(2-oxapentyl)-cyclohexyl]-ethan
1-(trans-4-Butylcyclohexyl)-2-[trans-4-(2-oxahexyl)-cyclohexyl]-ethan
1-(trans-4-Butylcyclohexyl)-2-[trans-4-(2-oxaheptyl)-cyclohexyl]-ethan
1-(trans-4-Pentylcyclohexyl)-2-[trans-4-(2-oxapropyl)-cyclohexyl]-ethan
1-(trans-4-Pentylcyclohexyl)-2-[trans-4-(2-oxabutyl)-cyclohexyl]-ethan
1-(trans-4-Pentylcyclohexyl)-2-[trans-4-(2-oxapentyl)-cyclohexyl]-ethan
1-(trans-4-Pentylcyclohexyl)-2-[trans-4-(2-oxahexyl)-cyclohexyl]-ethan
1-(trans-4-Pentylcyclohexyl)-2-[trans-4-(2-oxaheptyl)-cyclohexyl]-ethan
1-(trans-4-Propylcyclohexyl)-2-[trans-4-(2,5-dioxahexyl)-cyclohexyl]-ethan
1-(trans-4-Propylcyclohexyl)-2-[trans-4-(2,5-dioxaheptyl)-cyclohexyl]-ethan
1-(trans-4-Ethylcyclohexyl)-2-[trans-4-(2,5-dioxahexyl)-cyclohexyl]-ethan
1-(trans-4-Ethylcyclohexyl)-2-[trans-4-(2,5-dioxaheptyl)-cyclohexyl]-ethan
1-(trans-4-Butylcyclohexyl)-2-[trans-4-(2,5-dioxahexyl)-cyclohexyl]-ethan
1-(trans-4-Butylcyclohexyl)-2-[trans-4-(2,5-dioxaheptyl)-cyclohexyl]-ethan
1-(trans-4-Pentylcyclohexyl)-2-[trans-4-(2,5-dioxahexyl)-cyclohexyl]-ethan
1-(trans-4-Pentylcyclohexyl)-2-[trans-4-(2,5-dioxaheptyl)-cyclohexyl]-ethan

6. Ethanderivate der Teilformel 1a nach Anspruch 1, 2 oder 4 ausgewählt aus den folgenden Verbindungen:
1-(trans-4-Butylcyclohexyl)-2-[p-(1,4-dioxahexyl)-phenyl]-ethan
1-(trans-4-Butylcyclohexyl)-2-[p-(1,4-dioxaheptyl)-phenyl]-ethan
1-(trans-4-Butylcyclohexyl)-2-[p-(1,4-dioxapentyl)-phenyl]-ethan
1-(trans-4-Ethylcyclohexyl)-2-[p-(1,4-dioxahexyl)-phenyl]-ethan
1-(trans-4-Ethylcyclohexyl)-2-[p-(1,4-dioxaheptyl)-phenyl]-ethan
1-(trans-4-Propylcyclohexyl)-2-[p-(1,4-dioxahexyl)-phenyl]-ethan
1-(trans-4-Propylcyclohexyl)-2-[p-(1,4-dioxaheptyl)-phenyl]-ethan
1-(trans-4-Pentylcyclohexyl)-2-[p-(1,4-dioxahexyl)-pheny]-ethan
1-(trans-4-Pentylcyclohexyl)-2-[p-(1,4-dioxaheptyl)-phenyl]-ethan

7. Verwendung der Verbindungen nach einem der Ansprüche 1 bis 6 als Komponenten flüssigkristalliner Phasen.

8. Flüssigkristalline Phase mit mindestens zwei flüssigkristallinen Verbindungen, dadurch gekennzeichnet, daß sie mindestens eine Verbindung nach einem der Ansprüche 1 bis 6 enthält.

9. Flüssigkristall-Anzeigeelement, dadurch gekennzeichnet, daß es eine flüssigkristalline Phase nach Anspruch 8 enthält.

10. Elektrooptisches Anzeigeelement, dadurch gekennzeichnet, daß es als Dielektrikum eine flüssigkristalline Phase nach Anspruch 8 enthält.

## Claims

1. Ethane derivatives chosen from the formulae 1a and 1c 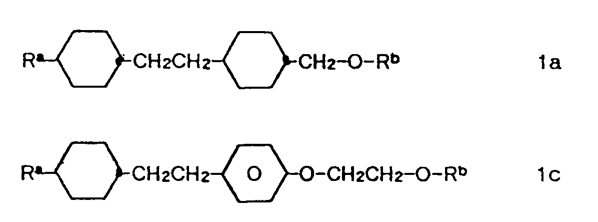 wherein
R^{a} is straight-chain alkyl having 1-8 C atoms and
R^{b} is straight-chain alkyl having 1-8 C atoms, wherein one or two non-adjacent and in each case non-terminal CH₂ group(s) can also be replaced by -O-.

2. Ethane derivatives according to Claim 1, wherein
R^{a} is straight-chain alkyl having 2-5 C atoms.

3. Ethane derivatives of the component formula 1a according to Claim 1 or 2, wherein
R^{b} is straight-chain alkyl having 1-5, in particular 1-3, C atoms.

4. Ethane derivatives of the component formula 1c according to Claim 1 or 2, wherein
R^{b} is straight-chain alkyl having 1-3 C atoms, in particular methyl or ethyl.

5. Ethane derivatives of the component formula 1a according to Claim 1, 2 or 3, chosen from the following compounds:
1-(trans-4-propylcyclohexyl)-2-[trans-4-(2-oxapropyl)-cyclohexyl]ethane
1-(trans-4-propylcyclohexyl)-2-[trans-4-(2-oxabutyl)-cyclohexyl]ethane
1-(trans-4-propylcyclohexyl)-2-[trans-4-(2-oxapentyl)-cyclohexyl]ethane
1-(trans-4-n-propylcyclohexyl)-2-[trans-4-(2-oxahexyl)-cyclohexyl]ethane
1-(trans-4-propylcyclohexyl)-2-[trans-4-(2-oxaheptyl)-cyclohexyl]ethane
1-(trans-4-ethylcyclohexyl)-2-[trans-4-(2-oxapropyl)-cyclohexyl]ethane
1-(trans-4-ethylcyclohexyl)-2-[trans-4-(2-oxabutyl)-cyclohexyl]ethane
1-(trans-4-ethylcyclohexyl)-2-[trans-4-(2-oxapentyl)-cyclohexyl]ethane
1-(trans-4-ethylcyclohexyl)-2-[trans-4-(2-oxahexyl)-cyclohexyl]ethane
1-(trans-4-ethylcyclohexyl)-2-[trans-4-(2-oxaheptyl)-cyclohexyl]ethane
1-(trans-4-butylcyclohexyl)-2-[trans-4-(2-oxapropyl)-cyclohexyl]ethane
1-(trans-4-butylcyclohexyl)-2-[trans-4-(2-oxabutyl)-cyclohexyl]ethane
1-(trans-4-butylcyclohexyl)-2-[trans-4-(2-oxapentyl)-cyclohexyl]ethane
1-(trans-4-butylcyclohexyl)-2-[trans-4-(2-oxahexyl)-cyclohexyl]ethane
1-(trans-4-butylcyclohexyl)-2-[trans-4-(2-oxaheptyl)-cyclohexyl]ethane
1-(trans-4-pentylcyclohexyl)-2-[trans-4-(2-oxapropyl)-cyclohexyl]ethane
1-(trans-4-pentylcyclohexyl)-2-[trans-4-(2-oxabutyl)-cyclohexyl]ethane
1-(trans-4-pentylcyclohexyl)-2-[trans-4-(2-oxapentyl)-cyclohexyl]ethane
1-(trans-4-pentylcyclohexyl)-2-[trans-4-(2-oxahexyl)-cyclohexyl]ethane
1-(trans-4-pentylcyclohexyl)-2-[trans-4-(2-oxaheptyl)-cyclohexyl]ethane
1-(trans-4-propylcyclohexyl)-2-[trans-4-(2,5-dioxahexyl)-cyclohexyl]ethane
1-(trans-4-propylcyclohexyl)-2-[trans-4-(2,5-dioxaheptyl)cyclohexyl]ethane
1-(trans-4-ethylcyclohexyl)-2-[trans-4-(2,5-dioxahexyl)-cyclohexyl]ethane
1-(trans-4-ethylcyclohexyl)-2-[trans-4-(2,5-dioxaheptyl)-cyclohexyl]ethane
1-(trans-4-butylcyclohexyl)-2-[trans-4-(2,5-dioxahexyl)-cyclohexyl]ethane
1-(trans-4-butylcyclohexyl)-2-[trans-4-(2,5-dioxaheptyl)-cyclohexyl]ethane
1-(trans-4-pentylcyclohexyl)-2-[trans-4-(2,5-dioxahexyl)-cyclohexyl]ethane
1-(trans-4-pentylcyclohexyl)-2-[trans-4-(2,5-dioxaheptyl)-cyclohexyl]ethane

6. Ethane derivatives of the component formula 1a according to Claim 1, 2 or 4, chosen from the following compounds:
1-(trans-4-butylcyclohexyl)-2-[p-(1,4-dioxahexyl)-phenyl]ethane
1-(trans-4-butylcyclohexyl)-2-[p-(1,4-dioxaheptyl)-phenyl]ethane
1-(trans-4-butylcyclohexyl)-2-[p-(1,4-dioxapentyl)-phenyl]ethane
1-(trans-4-ethylcyclohexyl)-2-[p-(1,4-dioxahexyl)-phenyl]ethane
1-(trans-4-ethylcyclohexyl)-2-[p-(1,4-dioxaheptyl)-phenyl]ethane
1-(trans-4-propylcyclohexyl)-2-[p-(1,4-dioxahexyl)-phenyl]ethane
1-(trans-4-propylcyclohexyl)-2-[p-(1,4-dioxaheptyl)-phenyl]ethane
1-(trans-4-pentylcyclohexyl)-2-[p-(1,4-dioxahexyl)-phenyl]ethane
1-(trans-4-pentylcyclohexyl)-2-[p-(1,4-dioxaheptyl)-phenyl]ethane

7. Use of the compounds according to one of Claims 1 to 6 as components of liquid-crystalline phases.

8. Liquid-crystalline phase with at least two liquid-crystalline compounds, characterized in that it comprises at least one compound according to one of Claims 1 to 6.

9. Liquid-crystal display element, characterized in that it comprises a liquid-crystalline phase according to Claim 8.

10. Electro-optical display element, characterized in that it comprises a liquid-crystalline phase according to Claim 8 as the dielectric.

## Revendications

1. Dérivés d'éthane choisis parmi les formules 1a et 1c, 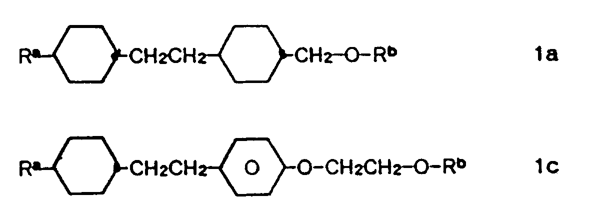 où
R^{a} représente un groupe alkyle en chaîne droite ayant 1-8 atomes de carbone, et
R^{b} désigne un groupe alkyle en chaîne droite ayant 1-8 atomes de carbone, dans lequel également un ou deux groupe(s) CH₂ non contigu(s) et pour chacun d'eux non terminal peu(ven)t être remplacé(s) par -O-.

2. Dérivés d'éthane selon la revendication 1 dans lesquels R^{a} désigne un groupe alkyle en chaîne droite ayant 2-5 atomes de carbone.

3. Dérivés d'éthane de formule de référence 1a selon l'une des revendications 1 ou 2, dans lesquels R^{b} désigne un groupe alkyle en chaîne droite ayant 1-5, en particulier 1-3, atomes de carbone.

4. Dérivés d'éthane de formule de référence 1c selon l'une des revendications 1 ou 2, dans lesquels R^{b} désigne un groupe alkyle en chaîne droite ayant 1-3 atomes de carbone, en particulier un groupe méthyle ou éthyle.

5. Dérivés d'éthane selon l'une quelconque des revendications 1, 2 ou 3, choisis parmi les composés suivants:
1-(trans-4-propylcyclohexyl)-2-[trans-4-(2-oxapropyl)-cyclohexyl]-éthane
1-(trans-4-propylcyclohexyl)-2-[trans-4-(2-oxabutyl)-cyclohexyl]-éthane
1-(trans-4-propylcyclohexyl)-2-[trans-4-(2-oxapentyl)-cyclohexyl]-éthane
1-(trans-4-n-propylcyclohexyl)-2-[trans-4-(2-oxahexyl)-cyclohexyl]-éthane
1-(trans-4-propylcyclohexyl)-2-[trans-4-(2-oxaheptyl)-cyclohexyl]-éthane
1-(trans-4-éthylcyclohexyl)-2-[trans-4-(2-oxapropyl)-cyclohexyl]-éthane
1-(trans-4-éthylcyclohexyl)-2-[trans-4-(2-oxabutyl)-cyclohexyl]-éthane
1-(trans-4-éthylcyclohexyl)-2-[trans-4-(2-oxapentyl)-cyclohexyl]-éthane
1-(trans-4-éthylcyclohexyl)-2-[trans-4-(2-oxahexyl)-cyclohexyl]-éthane
1-(trans-4-éthylcyclohexyl)-2-[trans-4-(2-oxaheptyl)-cyclohexyl]-éthane
1-(trans-4-butylcyclohexyl)-2-[trans-4-(2-oxapropyl)-cyclohexyl]-éthane
1-(trans-4-butylcyclohexyl)-2-[trans-4-(2-oxabutyl)-cyclohexyl]-éthane
1-(trans-4-butylcyclohexyl)-2-[trans-4-(2-oxapentyl)-cyclohexyl]-éthane
1-(trans-4-butylcyclohexyl)-2-[trans-4-(2-oxahexyl)-cyclohexyl]-éthane
1-(trans-4-butylcyclohexyl)-2-[trans-4-(2-oxaheptyl)-cyclohexyl]-éthane
1-(trans-4-pentylcyclohexyl)-2-[trans-4-(2-oxapropyl)-cyclohexyl]-éthane
1-(trans-4-pentylcyclohexyl)-2-[trans-4-(2-oxabutyl)-cyclohexyl]-éthane
1-(trans-4-pentylcyclohexyl)-2-[trans-4-(2-oxapentyl)-cyclohexyl]-éthane
1-(trans-4-pentylcyclohexyl)-2-[trans-4-(2-oxahexyl)-cyclohexyl]-éthane
1-(trans-4-pentylcyclohexyl)-2-[trans-4-(2-oxaheptyl)-cyclohexyl]-éthane
1-(trans-4-propylcyclohexyl)-2-[trans-4-(2,5-dioxahexyl)-cyclohexyl]-éthane
1-(trans-4-propylcyclohexyl)-2-[trans-4-(2,5-dioxaheptyl)-cyclohexyl]-éthane
1-(trans-4-éthylcyclohexyl)-2-[trans-4-(2,5-dioxahexyl)-cyclohexyl]-éthane
1-(trans-4-éthylcyclohexyl)-2-[trans-4-(2,5-dioxaheptyl)-cyclohexyl]-éthane
1-(trans-4-butylcyclohexyl)-2-[trans-4-(2,5-dioxahexyl)-cyclohexyl]-éthane
1-(trans-4-butylcyclohexyl)-2-[trans-4-(2,5-dioxaheptyl)-cyclohexyl]-éthane
1-(trans-4-pentylcyclohexyl)-2-[trans-4-(2,5-dioxahexyl)-cyclohexyl]-éthane
1-(trans-4-pentylcyclohexyl)-2-[trans-4-(2,5-dioxaheptyl)-cyclohexyl]-éthane.

6. Dérivés d'éthane de formule de référence 1a selon l'une quelconque des revendications 1, 2 ou 4, choisis parmi les composés suivants:
1-(trans-4-butylcyclohexyl)-2-[p-(1,4-dioxahexyl)-phényl]-éthane
1-(trans-4-butylcyclohexyl)-2-[p-(1,4-dioxaheptyl)-phényl]-éthane
1-(trans-4-butylcyclohexyl)-2-[p-(1,4-dioxapentyl)-phényl]-éthane
1-(trans-4-éthylcyclohexyl)-2-[p-(1,4-dioxahexyl)-phényl]-éthane
1-(trans-4-éthylcyclohexyl)-2-[p-(1,4-dioxaheptyl)-phényl]-éthane
1-(trans-4-propylcyclohexyl)-2-[p-(1,4-dioxahexyl)-phényl]-éthane
1-(trans-4-propylcyclohexyl)-2-[p-(1,4-dioxaheptyl)-phényl]-éthane
1-(trans-4-pentylcyclohexyl)-2-[p-(1,4-dioxahexyl)-phényl]-éthane
1-(trans-4-pentylcyclohexyl)-2-[p-(1,4-dioxaheptyl)-phényl]-éthane.

7. Utilisation des composés selon l'une quelconque des revendications 1 à 6 comme composants de phases de cristaux liquides.

8. Phase de cristaux liquides ayant au moins deux composés en cristaux liquides, caractérisée en ce qu'elle contient au moins un composé selon l'une quelconque des revendications 1 à 6.

9. Elément d'affichage, caractérisé en ce qu'il contient une phase de cristaux liquides selon la revendication 8.

10. Elément d'affichage électro-optique, caractérisé en ce qu'il contient comme diélectrique une phase de cristaux liquides selon la revendication 8.
